# EUROPEAN PATENT APPLICATION

(11) **EP 3 388 453 A1**
(43) Date of publication of application: **17.10.2018**
(21) Application number: 18166604.1
(22) Date of filing: 10.04.2018
(51) Int. Cl.: C08F 2/00, B01J 19/00, C08F 2/01, C08F 2/38, C08F 2/40

(54) **POLYMERIZATION INHIBITING SYSTEM AND POLYMERIZATION INHIBITING METHOD**

(30) Priority: 12.04.2017 JP 2017079053
(71) Applicant: Yokogawa Electric Corporation, Tokyo 180-8750 (JP)
(72) Inventor: Murata, Akihiro, Tokyo, 180-8750 (JP); Mitsumoto, Ken, Tokyo, 180-8750 (JP); Inoue, Takashi, Tokyo, 180-8750 (JP); Kurumado, Hiroshi, Kanagawa, 211-0053 (JP)
(74) Representative: Henkel, Breuer & Partner

(57) **Abstract**

A polymerization inhibiting system includes a measuring apparatus, a supplying apparatus and a control apparatus. The measuring apparatus is configured to continuously measure a concentration of an oligomer that is included in at least one of a raw material containing monomer as a main component and an intermediate product from the raw material in a manufacturing process for producing a product from the raw material. The supplying apparatus is configured to supply a polymerization inhibitor in the manufacturing process. The control apparatus is configured to control a supply rate of the polymerization inhibitor by the supplying apparatus, based on a measurement result of the measurement made by the measuring apparatus.

## Description

### BACKGROUND

### Technical Field

Disclosure of this application generally relates to a polymerization inhibiting system and a polymerization inhibiting method.

### Related Art

In a manufacturing process of a petroleum product or a chemical product, there are many cases where a step of manufacturing a polymer by causing a monomer or monomers to react in a polymerization process is performed. The term "a monomer" may hereinafter refer to a same type of monomer. The term "monomers" may hereinafter refer to monomers of two or more different types from or among each other. For example, the step of respectively manufacturing polyethylene, polystyrene, polyacrylonitrile, polybutadiene, polymethyl methacrylate (PMMA), or the like is performed by causing ethylene, styrene, acrylonitrile, butadiene, methyl methacrylate (MMA), or the like to be polymerized.

In the manufacturing process in which such a polymerization reaction is caused, it is known that a fouling (attached material) is generated. Here, the fouling in the manufacturing process of the petroleum product or the chemical product is referred to as a phenomenon that various materials are attached to an inner wall surface of an apparatus or piping.

A mechanism of the polymerization of a monomer which becomes a basis of the fouling generation is disclosed in "Ethylene Plant Cracker Gas Compressor Fouling", Sheri Snider, Technical Marketing, Nalco Company, Prepared for the AIChE Spring National Meeting, EPC Conference Houston, Texas April 23-26, 2006.

The mechanism is performed through a step of "Initiation" and a step of "Propagation" in sequence. Here, the step of "Initiation" is a step in which the monomer is changed into an unstable free radical by being excited due to heat. The step of "Propagation" is a step in which the free radical reacts with another monomer to form a new radical that is bonded to a dimer, a trimer, or the like (collectively referred to as an oligomer), and is grown to the polymer.

Meanwhile, since the monomer has a property of being likely to react, for example, in a process (such as a distillation process) before the process in which the original polymerization reaction is caused, or in the process for manufacturing the monomer, there is a problem that a portion of the monomer is polymerized against the intention to make the polymerization, thereby, the fouling is generated. If such a fouling is generated, for example, a production cost increases by deterioration in manufacturing efficiency of the product or lowering in working rate due to frequent maintenance.

Here, as described above, since the fouling is generated based on the polymerization of the monomer, in a process in which the generation of the fouling is wished to be prevented, if a polymerization inhibitor that hinders (or inhibits) the polymerization of the monomer is used, it is considered that it is possible to avoid the problems described above. However, in order to prevent the generation of the fouling using such a polymerization inhibitor, it greatly matters that progression of the polymerization is appropriately inhibited by continuously observing a polymerization state of an actual process which is changed from moment to moment, and controlling a supply rate of the polymerization inhibitor in accordance with an observation result. A methanol precipitation method is used as one example of a method for measuring the polymerization state of the actual process. The method is a method of measuring the polymerization state of the actual process by adding methanol to a collected sample, and measuring an amount (for example, a weight) of the polymer which is precipitated without being dissolved. Since the method is an off-line measurement which is performed at a frequency of approximately once a day, it is possible to measure the polymerization state of the actual process at a certain point of time, but it is difficult to measure the progression of the polymerization in the actual process which is changed from moment to moment.

In related art, the control to the supply rate is performed based on the actual measurements to the residual concentrations of the polymerization inhibitor, analysis to the amount of polymers, thermal transmittance, which can be difficult to perform precise or accurate control to the supply rate.

It is desirable to provide a polymerization inhibiting system and a polymerization inhibiting method that are capable of appropriately inhibiting progression of polymerization in an actual process which is changed from moment to moment.

### SUMMARY

A polymerization inhibiting system includes a measuring apparatus, a supplying apparatus and a control apparatus. The measuring apparatus is configured to continuously measure a concentration of an oligomer that is included in at least one of a raw material containing monomer as a main component and an intermediate product from the raw material in a manufacturing process for producing a product from the raw material. The supplying apparatus is configured to supply a polymerization inhibitor in the manufacturing process. The control apparatus is configured to control a supply rate of the polymerization inhibitor by the supplying apparatus, based on a measurement result of the measurement made by the measuring apparatus.

### BRIEF DESCRIPTION OF THE DRAWINGS

FIG. 1 is a block diagram illustrating a configuration of a main portion of a polymerization inhibiting system according to an embodiment of the present invention.
FIG. 2 is a diagram illustrating an example of information indicating a relationship between a supply rate of a polymerization inhibitor and a concentration of an oligomer which is used in the embodiment of the present invention.
FIG. 3 is a flowchart illustrating an example of an initial operation of the polymerization inhibiting system according to the embodiment of the present invention.
FIG. 4 is a flowchart illustrating an outline of an optimization operation of the polymerization inhibiting system according to the embodiment of the present invention.
FIG. 5 is a diagram for describing a difference between effects in a case where the optimization operation is performed and a case where the optimization operation is not performed in the embodiment of the present invention.

### DETAILED DESCRIPTION

In some aspects of the embodiments, a polymerization inhibiting system may include, but is not limited to, a measuring apparatus; a supplying apparatus; and a control apparatus. The measuring apparatus is configured to continuously measure a concentration of an oligomer that is included in at least one of a raw material containing monomer as a main component and an intermediate product from the raw material in a manufacturing process for producing a product from the raw material. The supplying apparatus is configured to supply a polymerization inhibitor in the manufacturing process. The control apparatus is configured to control a supply rate of the polymerization inhibitor by the supplying apparatus, based on a measurement result of the measurement made by the measuring apparatus.

In some cases, the control apparatus may be configured to store a relationship between the supply rate of the polymerization inhibitor and the concentration of the oligomer. The control apparatus is configured to control the supply rate of the polymerization inhibitor, using at least the measurement result and the relationship.

In some cases, the control apparatus is configured to store a respective relationship between the supply rate and the concentration for each process parameter set of different process parameters obtained in the manufacturing process. The control apparatus is configured to control the supply rate of the polymerization inhibitor, using the measurement result, each process parameter set of different process parameters, and the respective relationship for each process parameter set, to adjust the concentration of the oligomer to a predetermined concentration.

In some cases, the control apparatus is configured to control the supply rate of the polymerization inhibitor and the process parameters, using the measurement result, each process parameter set, and the respective relationship for each process parameter set.

In some cases, the control apparatus is configured to continuously control the supply rate of the polymerization inhibitor, using a latest one of the measurement results, each process parameter set of latest process parameters, and a latest one of the respective relationships for each process parameter set, to continuously adjust the concentration of the oligomer to the predetermined concentration.

In some cases, the measuring apparatus comprises: at least one of: an on-line gas chromatograph; and a Fourier transform infrared spectroscopy.

In other aspects of the embodiments, a polymerization inhibiting method may include, but is not limited to, continuously measuring a concentration of an oligomer that is included in at least one of a raw material containing monomer as a main component and an intermediate product from the raw material in a manufacturing process for producing a product from the raw material; supplying a polymerization inhibitor in the manufacturing process; and controlling a supply rate of the polymerization inhibitor, based on a measurement result of the measurement of the concentration of the oligomer.

In some cases, controlling the supply rate of the polymerization inhibitor may include, but is not limited to, controlling the supply rate of the polymerization inhibitor, using at least the measurement result of the measurement of the concentration of the oligomer and a relationship between the supply rate of the polymerization inhibitor and the concentration of the oligomer.

In some cases, controlling the supply rate of the polymerization inhibitor may include, but is not limited to, controlling the supply rate of the polymerization inhibitor, using the measurement result of the measurement of the concentration of the oligomer, each process parameter set of different process parameters, and a respective relationship between the supply rate and the concentration for each process parameter set of different process parameters obtained in the manufacturing process.

In some cases, controlling the supply rate of the polymerization inhibitor may include, but is not limited to, controlling the supply rate of the polymerization inhibitor and the process parameters, using the measurement result, each process parameter set, and the respective relationship for each process parameter set.

In some cases, controlling the supply rate of the polymerization inhibitor may include, but is not limited to, continuously controlling the supply rate of the polymerization inhibitor, using a latest one of the measurement results, each process parameter set of latest process parameters, and a latest one of the respective relationships for each process parameter set, to continuously adjust the concentration of the oligomer to a predetermined concentration.

In some cases, the concentration of the oligomer is continuously measured by at least one of: an on-line gas chromatograph; and a Fourier transform infrared spectroscopy.

The term "oligomer" in this context may refer to not only dimer and trimer, but also polymers. The term "supply rate of polymerization inhibitor" used herein may refer to a flow rate per unit time of polymerization inhibitor at which the polymerization inhibitor on a continuous flow of a fluid is supplied. The amount of supply of the polymerization inhibitor in the fluid may depend on the amount of radicals in the fluid. The amount of oligomer such as dimer can be the index of the amount of radial. In some cases, the concentration of oligomer in the fluid may be used to determine the amount of radical for controlling the supply rate of polymerization inhibitor. The concentration can be measured at ppm by either on-line gas chromatograph; or a Fourier transform infrared spectroscopy. The level of activity of the fluid depends on the amount of radical. The level of activity of the fluid also depends on operation conditions such as raw materials and physical quantities such as temperature. If the supply amount remains constant or fixed, the amount of oligomer should be changed or adjusted to the change of the operation conditions. It will be possible to perform a highly accurate control to the supply rate by adjusting to a variation of about 10% with reference to the actual concentration. It will also be possible to perform an accurate control to the supply rate by adjusting to a variation of about 50% with reference to the actual concentration.

The term "intermediate product" may refer to any product which is made from a raw material or materials and which is to be made into a final product. An example of the intermediate product may be methacrylic acids in case of methyl methacrylate monomer manufacturing apparatus. Any material such as DMF (dimethylformamide) in an extraction solvent may also be an intermediate product.

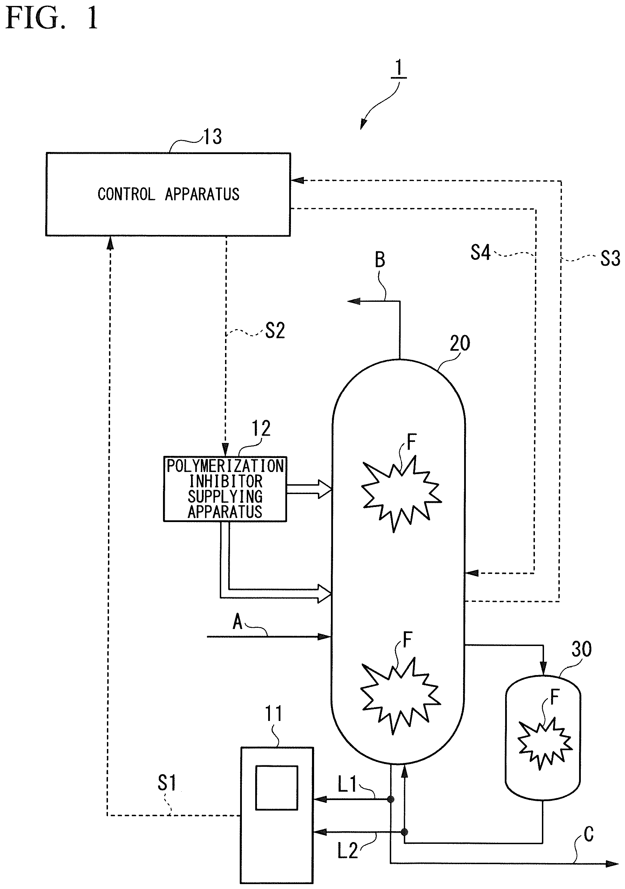

Hereinafter, a polymerization inhibiting system and a polymerization inhibiting method according to an embodiment of the present invention will be described in detail with reference to the drawings. FIG. 1 is a block diagram illustrating a configuration of a main portion of the polymerization inhibiting system according to the embodiment of the present invention. As illustrated in FIG. 1, a polymerization inhibiting system 1 according to the embodiment includes an on-line gas chromatograph 11 (measuring apparatus), a polymerization inhibitor supplying apparatus 12 (supply apparatus), and a control apparatus 13, and prevents generation of a fouling F within a rectifying tower 20 and a reboiler 30, by controlling a supply rate of a polymerization inhibitor which is supplied to the rectifying tower 20, and inhibiting polymerization of a monomer in the rectifying tower 20 and the reboiler 30.

Here, the rectifying tower 20 heats and gasifies a raw material A (raw material of which a main component is the monomer) that is supplied from the outside, and distills and separates the components that are included in the raw material A using a temperature difference within rectifying tower 20. For example, the raw material A is C4 (a mixture of hydrocarbon compounds including four carbon atoms), and the components that are distilled and separated in the rectifying tower 20 are B of acetylene or the like of which a boiling point is low, and C (a product) of butadiene of which boiling points are high. B of acetylene or the like is discharged to the outside from a tower head portion of the rectifying tower 20, and C of the butadiene is discharged (extracted) to the outside from a tower bottom portion of the rectifying tower 20. The reboiler 30 is disposed in order to supplement heating within the rectifying tower 20. The reboiler 30 heats the hydrocarbon compound which is supplied from the rectifying tower 20 to return the hydrocarbon compound into the rectifying tower 20.

The rectifying tower 20 and the reboiler 30 are facilities that are used in a manufacturing process in which C of the butadiene being the product is manufactured, by performing the component separation of the raw material A of which the main component is the monomer. The polymerization inhibiting system 1 prevents the generation of the fouling F by controlling the supply rate of the polymerization inhibitor that is supplied to the rectifying tower 20 being the facility which is used in such a manufacturing process, and inhibiting the polymerization of the monomer (butadiene) in the rectifying tower 20 and the reboiler 30. The fouling F is likely to be generated in the rectifying tower 20 and the reboiler 30 in which the heating is performed.

The on-line gas chromatograph 11 collects C of butadiene which is discharged to the outside from the tower bottom portion of the rectifying tower 20 and the hydrocarbon compound which is returned to the rectifying tower 20 from the reboiler 30, respectively, through collection lines L1 and L2, and continuously measures a concentration of an oligomer which is included in C of the collected butadiene and the collected hydrocarbon compound. For example, the on-line gas chromatograph 11 continuously measures the concentration of octadiene being a dimer of the butadiene. A measurement signal S1 indicating a measurement result of the on-line gas chromatograph 11 is output to the control apparatus 13.

The on-line gas chromatograph 11 includes a tube-shaped path that is referred to as a column, and a gas concentration detector that is disposed at an end edge of the column. The on-line gas chromatograph 11 separates the gas of a measurement target per component in accordance with a difference in the time at which the column is moved, and measures the concentration per component which is included in the gas of the measurement target by detecting the separated component with the gas concentration detector. In such an on-line gas chromatograph 11, for example, the concentration measurement is performed at intervals of several minutes to several tens of minutes. For example, the measurement signal S1 indicating the measurement result is continuously output at intervals of several minutes to several tens of minutes. The on-line gas chromatograph 11 may continuously perform the concentration measurement, and continuously output the measurement signal S1 indicating the measurement result.

The polymerization inhibitor supplying apparatus 12 supplies the polymerization inhibitor to an inside of the rectifying tower 20, under the control of the control apparatus 13. Specifically, the polymerization inhibitor supplying apparatus 12 supplies the polymerization inhibitor as an amount represented by a control signal S2 which is output from the control apparatus 13. Here, as a polymerization inhibitor which is supplied to the inside of the rectifying tower 20 by the polymerization inhibitor supplying apparatus 12, for example, it is possible to use 4-hydroxy TEMPO, 4-tertiary butyl catechol, or the like which is a stable free radical (SFR).

The control apparatus 13 controls the supply rate of the polymerization inhibitor which is supplied to the inside of the rectifying tower 20 by the polymerization inhibitor supplying apparatus 12, based on the measurement result represented by the measurement signal S1 which is output from the on-line gas chromatograph 11. The control apparatus 13 controls the supply rate of the polymerization inhibitor with respect to the rectifying tower 20, by outputting the control signal S2 to the polymerization inhibitor supplying apparatus 12. Here, the control apparatus 13 stores information indicating a relationship between the supply rate of the polymerization inhibitor and the concentration of the octadiene (oligomer) in advance, and controls the supply rate of the polymerization inhibitor, using the measurement result represented by the measurement signal S1 which is output from the on-line gas chromatograph 11 and the information. For example, the information may be information of a table form, or information represented by a function.

More specifically, the control apparatus 13 stores the information indicating the relationship between the supply rate of the polymerization inhibitor and the concentration of the octadiene (oligomer) per combination of a plurality of process parameters which are different from each other. The process parameters are referred to as a temperature, a pressure, a flow rate, and the like in the manufacturing process. The control apparatus 13 is capable of obtaining the process parameters from a detection signal S3 indicating a detection result of a sensor (such as a temperature sensor, a pressure sensor, or a flow rate sensor) that is disposed in the rectifying tower 20 and is not illustrated in the drawing. The control apparatus 13 is capable of performing the control of the process parameters, by outputting a control signal S4 for controlling an operation condition to the rectifying tower 20 (or the rectifying tower 20 and the reboiler 30).

The control apparatus 13 controls the supply rate of the polymerization inhibitor, using the measurement result that is represented by the measurement signal S1 which is outputted from the on-line gas chromatograph 11, the process parameter that is obtained from the detection signal S3, and the information per combination of the process parameters. For example, the control apparatus 13 controls the supply rate of the polymerization inhibitor such that the concentration of the octadiene (oligomer) becomes a predefined concentration. The control apparatus 13 may perform the control of the process parameter, in addition to the control of the supply rate of the polymerization inhibitor.

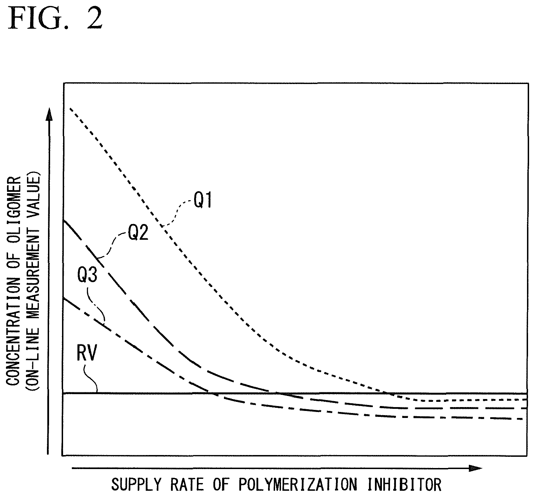

FIG. 2 is a diagram illustrating an example of the information indicating the relationship between the supply rate of the polymerization inhibitor and the concentration of the oligomer which are used in the embodiment of the present invention. In a graph illustrated in FIG. 2, a horizontal axis represents the supply rate of the polymerization inhibitor, and a vertical axis represents the concentration of the oligomer (the concentration of the octadiene which is measured in an on-line manner by the on-line gas chromatograph 11). Curved lines to which marks of Q1 to Q3 are attached in FIG. 2 are the information indicating the relationship between the supply rate of the polymerization inhibitor and the concentration of the octadiene (oligomer). The curved lines of Q1 to Q3 are the combinations of the plurality of process parameters which are different from each other. For example, the curved lines of Q1 to Q3 indicate the relationship between the supply rate of the polymerization inhibitor and the concentration of the octadiene (oligomer) in a case where the pressure and the flow rate are the same, but the temperatures are different from each other.

Referring to FIG. 2, it is understood that any of the curved lines of Q1 to Q3 indicates a right-sided downward change although variation is present somewhat in accordance with the combination of the process parameters. It is considered that a degree of inhibiting the polymerization of the monomer becomes large as the supply rate of the polymerization inhibitor increases, thereby, the concentration of the octadiene (oligomer) is lowered. However, if the supply rate of the polymerization inhibitor becomes large to a certain extent, it is understood that the concentration of the octadiene (oligomer) is not changed much even in a case where the supply rate of the polymerization inhibitor increases to the certain extent or more. There is a need to avoid the further supply of the polymerization inhibitor in a case where the supply rate of the polymerization inhibitor becomes large to a certain extent since it is conceivable that the further supply becomes a surplus supply.

Next, an operation of the polymerization inhibiting system 1 in the above configuration will be described. The operations of the polymerization inhibiting system 1 are broadly classified into two operations. The first one is an operation (referred to as an "initial operation", hereinafter) at the time of obtaining the information indicating the relationship between the supply rate of the polymerization inhibitor and the concentration of the octadiene (oligomer). The second one is an operation (referred to as an "optimization operation", hereinafter) at the time of appropriately inhibiting progression of the polymerization of the monomer. Hereinafter, the initial operation will be described, and subsequently, the optimization operation will be described.

### Initial Operation

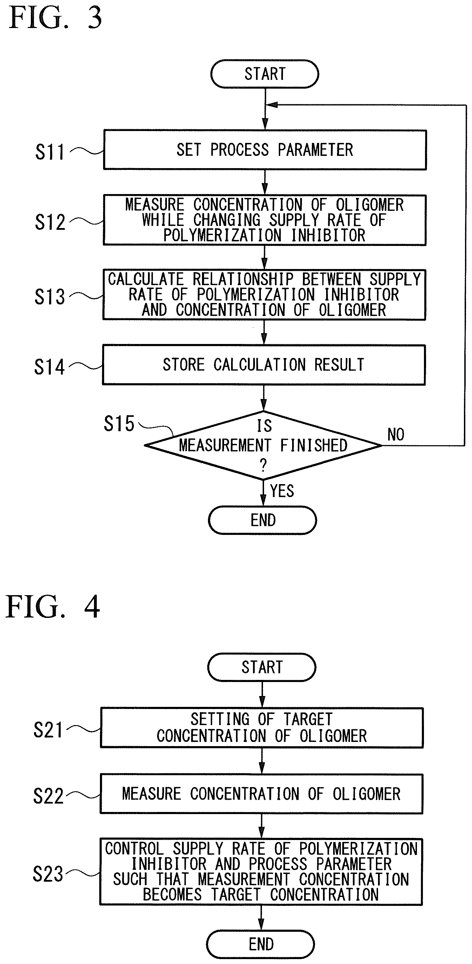

FIG. 3 is a flowchart illustrating an example of the initial operation of the polymerization inhibiting system according to the embodiment of the present invention. If the initial operation is started, a treatment of setting the process parameter (such as the temperature, the pressure, or the flow rate), based on an instruction that is supply from a supply apparatus which is not illustrated in the drawing, is performed by the control apparatus 13 (step S11). If the setting is performed, the control signal S4 is output to the rectifying tower 20 (or the rectifying tower 20 and the reboiler 30) from the control apparatus 13, and the operation condition of the rectifying tower 20 or the like is controlled such that the process parameter (process parameter which is obtained from the detection signal S3) which is detected in the rectifying tower 20 becomes the set process parameter.

Next, the treatment of measuring the concentration of the octadiene (oligomer), while changing the supply rate of the polymerization inhibitor, is performed (step S12). Specifically, the treatment of sequentially obtaining the measurement result represented by the measurement signal S1 which is output from the on-line gas chromatograph 11, while changing the supply rate of the polymerization inhibitor by sequentially outputting the control signal S2 to the polymerization inhibitor supplying apparatus 12, is performed by the control apparatus 13.

Subsequently, the treatment of calculating the relationship between the supply rate of the polymerization inhibitor and the concentration of the octadiene (oligomer) is performed (step S13). For example, the treatment of making a table in which the supply rate of the polymerization inhibitor represented by the control signal S2 and the concentration of the octadiene (oligomer) represented by the measurement signal S1 are associated with each other one to one, or the treatment of calculating an approximate equation indicating the relationship between the supply rate of the polymerization inhibitor and the concentration of the octadiene (oligomer) is performed. Therefore, the treatment of storing the calculated relationship is performed by the control apparatus 13 (step S14).

If the above treatments are finished, the control apparatus 13 determines whether or not the measurement treatment (treatment of measuring the concentration of the octadiene (oligomer) while changing the supply rate of the polymerization inhibitor) described above is finished (step S15). In a case where the measurement treatment described above is determined to be not finished (in a case where the determination result of the step S15 is "NO"), the steps of S11 to S 14 are performed again. That is, the treatment of changing the process parameter, and calculating the relationship between the supply rate of the polymerization inhibitor and the concentration of the octadiene (oligomer) to store the relationship is performed.

On the contrary, in a case where the measurement treatment described above is determined to be finished (in a case where the determination result of the step S15 is "YES"), a series of treatments illustrated in FIG. 3 are finished. By performing the above treatments, the information (see FIG. 2) indicating the relationship between the supply rate of the polymerization inhibitor and the concentration of the octadiene (oligomer) per combination of the process parameters is stored in the control apparatus 13.

### Optimization Operation

FIG. 4 is a flowchart illustrating an outline of the optimization operation of the polymerization inhibiting system according to the embodiment of the present invention. If the optimization operation is started, first, the treatment of setting a target concentration RV (see FIG. 2) of the octadiene (oligomer), based on the instruction that is supply from the supplying apparatus which is not illustrated in the drawing, is performed by the control apparatus 13 (step S21). For example, the target concentration RV is set to be a concentration that is expected to be capable of preventing the generation of the fouling F from past experience.

Next, the treatment of continuously measuring the concentration of the octadiene (oligomer) is performed, using the on-line gas chromatograph 11 (step S22: a first step). As described above, in the on-line gas chromatograph 11, for example, the concentration measurement is performed at intervals of several minutes to several tens of minutes. Therefore, for example, the measurement signal S1 indicating the measurement result of the concentration of the octadiene (oligomer) is continuously supply to the control apparatus 13 at intervals of several minutes to several tens of minutes.

If the concentration of the octadiene (oligomer) is measured, the treatment of controlling the supply rate of the polymerization inhibitor and the process parameter such that the measurement concentration becomes the target concentration RV, is performed by the control apparatus 13 (step S23: a second step and a third step). Specifically, the supply rate of the polymerization inhibitor is controlled using the measurement result that is represented by the measurement signal S1 which is output from the on-line gas chromatograph 11, the process parameter that is obtained from the detection signal S3, and the information (the information illustrated in FIG. 2) per combination of the process parameters, thereby, the measurement concentration becomes the target concentration RV.

Here, in an actual process, for example, the process parameter may vary in accordance with the amount of impurities which are included in the raw material A. In a case where the variation of the process parameter is generated, the control apparatus 13 controls the supply rate of the polymerization inhibitor, or the like using the information which is suitable for the process parameter in the stored information. For example, in a case where the information having the relationship represented by the curved line Q1 in FIG. 2 is used, if the variation of the process parameter is generated, the supply rate of the polymerization inhibitor is controlled using the information (for example, the information having the relationship represented by the curved line Q2) which is suitable for the process parameter after the variation.

By performing the above control, it is possible to appropriately inhibit the progression of the polymerization in the actual process that is changed from moment to moment. In FIG. 4, an example in which the step (step S23) is performed after the measurement (step S22) of the concentration of the octadiene (oligomer) is illustrated, but the treatment of the step S22 and the treatment of the step S23 may be performed in parallel. That is, the treatment of controlling the supply rate of the polymerization inhibitor or the like such that the measurement concentration becomes the target concentration RV, while performing the measurement of the concentration of the octadiene (oligomer), may be performed.

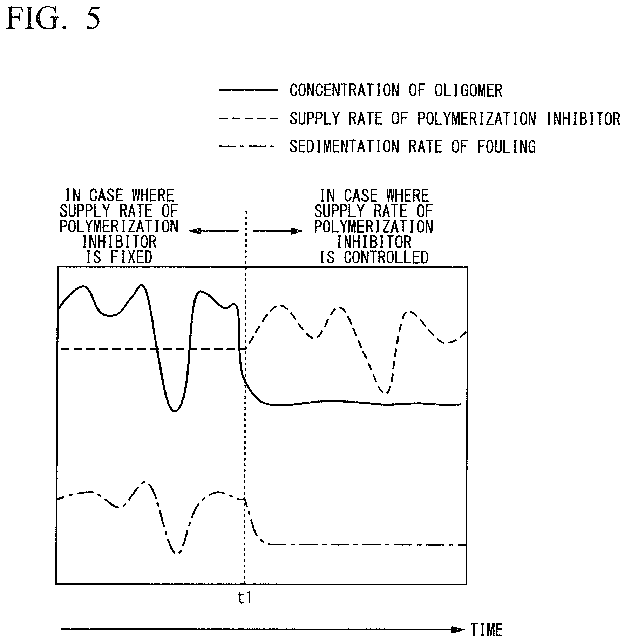

FIG. 5 is a diagram for describing the difference between effects in a case where the optimization operation is performed and a case where the optimization operation is not performed in the embodiment of the present invention. In FIG. 5, the horizontal axis represents the time, and the vertical axis represents the concentration of the oligomer, the supply rate of the polymerization inhibitor, and a sedimentation rate of the fouling F. In FIG. 5, a time-dependent change of the concentration of the oligomer is represented by a solid line, the time-dependent change of the supply rate of the polymerization inhibitor is represented by a broken line, and the time-dependent change of the sedimentation rate of the fouling F is represented by a dashed line. In FIG. 5, the optimization operation is not performed until a time t1, and the optimization operation is performed from the time t1.

As illustrated in FIG. 5, since the optimization operation is not performed until the time t1, the supply rate of the polymerization inhibitor is fixed until the time t1. Referring to FIG. 5, it is understood that the concentration of the oligomer greatly varies until the time t1 at which the optimization operation is not performed, and the sedimentation rate of the fouling F greatly varies in accordance therewith. In general, a way of the variation of the sedimentation rate of the fouling F is the same as the way of the variation of the concentration of the oligomer. That is, there is a tendency that the sedimentation rate of the fouling F generally increases if the concentration of the oligomer increases, and the sedimentation rate of the fouling F is generally dropped if the concentration of the oligomer is lowered.

If the optimization operation is started at the time t1, since the supply rate of the polymerization inhibitor is controlled by the control apparatus 13, the supply rate of the polymerization inhibitor varies, as illustrated in FIG. 5. Referring to FIG. 5, it is understood that the concentration of the oligomer and the sedimentation rate of the fouling F are substantially fixed after the time t1 at which the optimization operation is started. Here, since the sedimentation rate of the fouling F after the time t1 at which the optimization operation is started is lowered on the whole in comparison with the sedimentation rate of the fouling F before the time t1, it is understood that the generation of the fouling F is prevented, by performing the optimization operation.

As described above, in the embodiment, since the concentration of octadiene (oligomer) is continuously measured using the on-line gas chromatograph 11, and the supply rate of the polymerization inhibitor and the process parameter are controlled such that the measurement concentration becomes the target concentration RV, it is possible to appropriately inhibit the progression of the polymerization in the actual process that is changed from moment to moment. Thereby, it is possible to prevent the generation of the fouling F, and it is possible to prevent a cost increase that is caused by, for example, deterioration in manufacturing efficiency of the product or lowering in working rate due to frequent maintenance.

In the embodiment, the control of the supply rate of the polymerization inhibitor or the like is performed, using the information indicating the relationship between the supply rate of the polymerization inhibitor and the concentration of the octadiene (oligomer) that are prepared per combination of the plurality of process parameters which are different from each other. Therefore, a lack of supply of the polymerization inhibitor and the excess supply thereof are prevented, thereby, it is possible to appropriately inhibit the progression of the polymerization in the actual process that is changed from moment to moment with the polymerization inhibitor of the suitable amount.

Hereupon, the polymerization inhibiting system and the polymerization inhibiting method according to the embodiment of the present invention are described, but the present invention is not limited to the embodiments described above, and it is possible to freely modify the embodiments within the scope of the present invention. For example, in the embodiment described above, in order to be easily understood, an example in which only the concentration of the octadiene being one of the oligomers is measured by the on-line gas chromatograph 11 is described. However, in a case where a plurality of components are included in the oligomer, only one of the plurality of components may be measured, or the plurality of components may be measured.

In the embodiments described above, the concentration of the octadiene (oligomer) is measured using the on-line gas chromatograph 11, but the measurement of the concentration may be performed using a spectroscopic analysis apparatus such as a Fourier transform infrared spectroscopy (FT-IR), in replacement of the on-line gas chromatograph 11. The concentration measurement of the on-line manner may be performed using a so-called hard sensor such as the on-line gas chromatograph 11 or the spectroscopic analysis apparatus, or the concentration measurement of the on-line manner may be performed using a soft sensor.

In the embodiments described above, an example in which the supply rate of the polymerization inhibitor and the process parameter are controlled such that the measurement concentration of the on-line gas chromatograph 11 becomes the target concentration RV, is described. However, both of the supply rate of the polymerization inhibitor and the process parameter are not necessarily controlled, and only one thereof may be controlled. In the embodiments described above, the manufacturing process in which the butadiene are manufactured is described, but the gist thereof is not the gist of being limited to the manufacturing process in which butadiene are manufactured, and it is possible to apply the manufacturing process to the manufacturing process in which the optional product is manufactured from the raw material of which the main component is the monomer.

While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.

## Claims

1. A polymerization inhibiting system comprising:
a measuring apparatus (11) configured to continuously measure a concentration of an oligomer that is included in at least one of a raw material containing monomer as a main component and an intermediate product from the raw material in a manufacturing process for producing a product from the raw material;
a supplying apparatus (12) configured to supply a polymerization inhibitor in the manufacturing process, and
**characterized by** further comprising:
a control apparatus (13) configured to control a supply rate of the polymerization inhibitor by the supplying apparatus (12), based on a measurement result of the measurement made by the measuring apparatus (11).

2. The polymerization inhibiting system according to Claim 1,
**characterized in that** the control apparatus (13) is configured to store a relationship between the supply rate of the polymerization inhibitor and the concentration of the oligomer, and
wherein the control apparatus (13) is configured to control the supply rate of the polymerization inhibitor, using at least the measurement result and the relationship.

3. The polymerization inhibiting system according to Claim 2,
**characterized in that** the control apparatus (13) is configured to store a respective relationship between the supply rate and the concentration for each process parameter set of different process parameters obtained in the manufacturing process, and
that the control apparatus (13) is configured to control the supply rate of the polymerization inhibitor, using the measurement result, each process parameter set of different process parameters, and the respective relationship for each process parameter set, to adjust the concentration of the oligomer to a predetermined concentration.

4. The polymerization inhibiting system according to Claim 3,
**characterized in that** the control apparatus (13) is configured to control the supply rate of the polymerization inhibitor and the process parameters, using the measurement result, each process parameter set, and the respective relationship for each process parameter set.

5. The polymerization inhibiting system according to Claim 3,
**characterized in that** the control apparatus (13) is configured to continuously control the supply rate of the polymerization inhibitor, using a latest one of the measurement results, each process parameter set of latest process parameters, and a latest one of the respective relationships for each process parameter set, to continuously adjust the concentration of the oligomer to the predetermined concentration.

6. The polymerization inhibiting system according to Claim 1,
**characterized in that** the measuring apparatus (11) comprises: at least one of: an on-line gas chromatograph; and a Fourier transform infrared spectroscopy.

7. A polymerization inhibiting method comprising:
continuously measuring a concentration of an oligomer that is included in at least one of a raw material containing monomer as a main component and an intermediate product from the raw material in a manufacturing process for producing a product from the raw material;
supplying a polymerization inhibitor in the manufacturing process; and
**characterized by** further comprising:
controlling a supply rate of the polymerization inhibitor, based on a measurement result of the measurement of the concentration of the oligomer.

8. The polymerization inhibiting method according to Claim 7,
**characterized in that** controlling the supply rate of the polymerization inhibitor comprises controlling the supply rate of the polymerization inhibitor, using at least the measurement result of the measurement of the concentration of the oligomer and a relationship between the supply rate of the polymerization inhibitor and the concentration of the oligomer.

9. The polymerization inhibiting method according to Claim 8,
**characterized in that** controlling the supply rate of the polymerization inhibitor comprises controlling the supply rate of the polymerization inhibitor, using the measurement result of the measurement of the concentration of the oligomer, each process parameter set of different process parameters, and a respective relationship between the supply rate and the concentration for each process parameter set of different process parameters obtained in the manufacturing process.

10. The polymerization inhibiting method according to Claim 9,
**characterized in that** controlling the supply rate of the polymerization inhibitor comprises controlling the supply rate of the polymerization inhibitor and the process parameters, using the measurement result, each process parameter set, and the respective relationship for each process parameter set.

11. The polymerization inhibiting method according to Claim 9,
**characterized in that** controlling the supply rate of the polymerization inhibitor comprises continuously controlling the supply rate of the polymerization inhibitor, using a latest one of the measurement results, each process parameter set of latest process parameters, and a latest one of the respective relationships for each process parameter set, to continuously adjust the concentration of the oligomer to a predetermined concentration.

12. The polymerization inhibiting method according to Claim 7,
**characterized in that** the concentration of the oligomer is continuously measured by at least one of: an on-line gas chromatograph; and a Fourier transform infrared spectroscopy.
